(19) Europäisches Patentamt
European Patent Office
Office européen des brevets

(11) **EP 1 927 287 B1**

(12) **EUROPEAN PATENT SPECIFICATION**

(45) Date of publication and mention
of the grant of the patent:
**27.04.2016 Bulletin 2016/17**

(51) Int Cl.:
*A23D 7/01* (2006.01)   *A23L 2/58* (2006.01)
*A61Q 19/10* (2006.01)   *A23L 5/44* (2016.01)
*B01F 17/00* (2006.01)   *A23D 7/00* (2006.01)

(21) Application number: **07023299.6**

(22) Date of filing: **30.11.2007**

(54) **EMULSION COMPOSITION, AND FOODS AND COSMETICS CONTAINING THE EMULSION COMPOSITION**

EMULSIONSZUSAMMENSETZUNG SOWIE LEBENSMITTEL UND KOSMETIKARTIKEL MIT DER EMULSIONSZUSAMMENSETZUNG

COMPOSITION D'ÉMULSION, ET ALIMENTS ET COSMÉTIQUES CONTENANT LA COMPOSITION D'ÉMULSION

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **01.12.2006 JP 2006325642**
**06.09.2007 JP 2007231675**

(43) Date of publication of application:
**04.06.2008 Bulletin 2008/23**

(73) Proprietor: **FUJIFILM Corporation**
**Minato-ku**
**Tokyo (JP)**

(72) Inventors:
• **Ogawa, Manabu**
**Kaisei-machi,**
**Ashigarakami-gun**
**Kanagawa (JP)**
• **Arakawa, Jun**
**Kaisei-machi,**
**Ashigarakami-gun**
**Kanagawa (JP)**
• **Suzuki, Keiichi**
**Kaisei-machi,**
**Ashigarakami-gun**
**Kanagawa (JP)**
• **Nagata, Kozo**
**Minato-ku**
**Tokyo (JP)**

(74) Representative: **Höhfeld, Jochen et al**
**Klunker Schmitt-Nilson Hirsch**
**Patentanwälte**
**Destouchesstrasse 68**
**80796 München (DE)**

(56) References cited:
**EP-A- 1 281 396      EP-A- 1 474 992**
**WO-A-95/28847      JP-A- 2006 129 841**
**US-A- 5 248 509**

• **DATABASE WPI Week 199734 Thomson Scientific, London, GB; AN 1997-369373 XP002472920 & JP 09 157159 A (LION CORP) 17 June 1997 (1997-06-17)**
• **DATABASE WPI Week 199536 Thomson Scientific, London, GB; AN 1995-271580 XP002472921 & JP 07 171377 A (SANEIGEN FFI KK) 11 July 1995 (1995-07-11)**

**Description**

BACKGROUND OF THE INVENTION

Field of the Invention

The present invention relates to an emulsion composition, and foodstuff and cosmetics containing the same.

Description of the Related Art

Conventionally, oil-based components have been added to aqueous beverages, aqueous foodstuff and aqueous cosmetics. However, since oil-based components are insoluble or are sparingly soluble in water, oil-based components have generally been dispersed in an aqueous medium as an emulsion by emulsification or the like. Since emulsions scatter light, depending on their particle size, therefore, when an emulsion has a large particle size, turbidity occurs in the emulsion, together with foodstuff and cosmetics having the emulsion added thereto, which may be not preferable in terms of appearance. It has therefore been desired to make the particle size of emulsions fine enough such that the scattering of light is considerably diminished.

Additionally, emulsions are generally in a semi-stable state, and there are considerable problems with the particle size increasing during storage and with water phase and oil phase separation when an emulsion is stored over a long period of time. Adhesion of congealed oil droplets to the walls or ring of the neck of containers in beverages is an example of such an oil droplet separation phenomenon in such an emulsion.

Regarding the improvement of the above-described transparency and stability with time, a method of uniformly dispersing an amphiphilic substance such as lecithin and a surfactant in water and then emulsifying is disclosed (see, for example, JP-A No. 2-78432).

Furthermore, regarding improvements to the above-described transparency and stability with time, a nano-emulsion using a surfactant which is a sugar ester or ether that is solid at 45°C or lower, and at least one oil having a molecular weight exceeding 400, the weight ratio of the amount of oil phase being in a range of from 2 to 10 times the amount of the surfactant, is disclosed (see, for example, JP-A No. 2000-178130).

Additionally, an emulsion-like water-soluble condensate wherein the proportion and concentration of lecithin and lipid, and the concentration of a polyol solution contained are regulated is disclosed (see, for example, JP-T No. 2006-513172) (the term "JP-T" as used herein means a published Japanese translation of a PCT patent application). Similar formulations are disclosed in JP 09157159A, JP07171377A, WO 95/28847A, EP 1474992A, JP2006129841, EP 1281396Aand US 5,248,509

However, even with the technologies of the above patent documents the particle size of emulsified particles and emulsion stability of the emulsion composition is not yet satisfactory.

SUMMARY OF THE INVENTION

The present invention has been made in view of the above circumstances and provides an emulsion composition, and foodstuff and cosmetics containing the emulsion composition.

A first aspect of the present invention provides an emulsion composition including a phospholipid, a surfactant and an oil-based component comprising astaxanthin or a derivative thereof, wherein the content of the surfactant exceeds 0.5 times by weight but does not exceed 2 times by weight the content of the oil-based component, and exceeds 5 times by weight but does not exceed 50 times by weight the content of the phospholipid and wherein the amount of surfactant present is 2 - 15% by mass based on the mass of the emulsion composition.

DETAILED DESCRIPTION OF THE INVENTION

An object of the invention is to provide an emulsion composition having a small particle size of emulsified particles and excellent emulsion stability.

A further object of the invention is to provide foodstuff and cosmetics containing an emulsion composition having a small particle size of emulsified particles and excellent emulsion stability.

As a result of extensive and intensive investigations in view of the above circumstances, it has been found that the above problems can be overcome, and the invention has been completed. The invention is achieved by the following means.

The emulsion composition of the invention includes a phospholipid, a surfactant and an oil-based component comprising astaxanthin or a derivative thereof, wherein the content of the surfactant exceeds 0.5 times by weight but does not exceed 2 times by weight the content of the oil-based component, and exceeds 5 times by weight but does not exceed 50 times by weight the content of the phospholipid and wherein the amount of surfactant present is 2 - 15% by mass based on the mass of the emulsion composition.

Thus, when the amount of the surfactant in the emulsion composition is adjusted to the above range, based on the content of the oil-based component and the content of the phospholipid, the particle size of the emulsified particles can be decreased, and additionally the emulsion stability can be made good.

The emulsion composition of the invention is an oil-in-water emulsion, and each constituent contained in the oil phase and the water phase is described below.

[Phospholipid]

Phospholipid contained in the emulsion composition of the invention is an ester comprising, in a complex lipid, a fatty acid, an alcohol, phosphorus and a nitrogen compound, is a group having a phosphoric acid ester and a fatty acid ester, and means a glycerophospholipid and a sphingophospholipid. The phospholipid is described in detail below.

Examples of glycerophospholipids that can be used as the phospholipid in the invention include phosphatidic acid, bisphosphatidic acid, lecithin (phosphatidylcholine), phosphatidylethanolamine, phosphatidylmethylethanolamine, phosphatidylserin, phosphatidylinositol, phosphatidylglycerin and diphosphatidylglycerin (cardiolipin), and additionally include various lecithins including those derived from plants such as soybeans, corn, peanuts, rapeseeds and wheat like cereals, various lecithins derived from animals such as egg yolk and cows, and various lecithins derived from microorganisms such as Escherichia coli.

Sphingophospholipids that may be used as the phospholipid in the invention include, for example, sphingomyelin.

Furthermore, the invention can use enzyme-decomposed glycerophospholipid as the glycerophospholipid. For example, lysolecithin (enzyme decomposed lecithin) formed by enzyme decomposition of lecithin so that a fatty acid (acyl group) bonded to 1-position and/or to the 2-position of glycerophospholipid is lost. When only one fatty acid group is present, hydrophilicity of lecithin can be improved, and emulsifiability and dispersibility in water can be improved.

Lysolecithin is obtained by hydrolysis of lecithin in the presence of an acid or alkali catalyst, but can also be obtained by hydrolysis of lecithin using phospholipase $A_1$ or $A_2$.

Examples that may be given of the lyso compound represented by such a lysolecithin include lisophosphatidic acid, lysophosphatidylglycerin, lysophosphatidylinositol, lysophosphatidylethanolamine, lysophosphatidylmethylethanolamine, lysophosphatidylcholine (lysolecithin) and lysophosphatidylserine.

Furthermore, the glycerophospholipid represented by the lecithin in the invention can use hydrogenated or hydroxylated products.

Hydrogenation can be carried out by, for example, reacting lecithin with hydrogen in the presence of a catalyst, thereby the unsaturated bond in the fatty acid moiety is hydrogenated. The hydrogenation improves oxidation stability of lecithin.

Hydroxylation is where unsaturated bond(s) in the fatty acid moiety are hydroxylated by heating lecithin together with high concentration of hydrogen peroxide and an organic acid such as acetic acid, tartaric acid or butyric acid. Hydroxylation improves the hydrophilicity of lecithin.

Hydrogenated or hydroxylated lecithins are particularly preferred for applications in cosmetics.

Of the above lipids, lecithin and lysolecithin as the glycerophospholipid are preferred from the point of the emulsion stability, and lecithin is more preferred.

Lecithin has a hydrophilic group and a hydrophobic group in the molecule, and is therefore conventionally widely used as an emulsifier in the fields of foodstuff, medicines and cosmetics.

Lecithin having a purity of 60% by mass or more is industrially used as lecithin. In the invention, lecithin having a purity of 80% by mass or more generally called "high purity lecithin" is preferred, and lecithin having a purity of 90% by mass or more is more preferred.

The lecithin purity (% by mass) is obtained by subtracting the weights of toluene-insoluble substances and acetone-soluble substances, utilizing the property that lecithin tends to dissolve in toluene and does not dissolve in acetone.

High purity lecithin has high lipophilicity as compared with lysolecithin, and it is therefore considered that compatibility between lecithin and an oil-based component is increased, thereby improving emulsion stability.

The phospholipid used in the invention can be used alone or as mixtures of two or more thereof.

In the emulsion composition of the invention, the content of the phospholipid is preferably from 0.1% to 10% by mass, more preferably from 0.2% to 5% by mass, and further preferably from 0.5% to 2% by mass, based on the mass of the total composition.

When the content of the phospholipid is 0.1% by mass or more, there is the tendency for the emulsion stability of the emulsion composition to be good. Furthermore, when the content is 10% by mass or less, phospholipid dispersion is not formed in water by separating excess phospholipid from an oil-based component, and this is therefore preferred

3

from the point of emulsion stability of the emulsion composition.

[Oil-based component]

The oil-based component used in the emulsion composition of the invention is described below.

Oil-based components that can be used in the invention are not particularly limited so long as they are components that do not dissolve in an aqueous medium but do dissolve in an oil-based medium. Radical trapping agents containing oil-soluble vitamins, such as carotenoids and tocopherols, and oils and fats such as coconut oil are preferably used.

The carotenoids used in the invention are astaxanthin and/or its derivatives such as esters of astaxanthin (hereinafter generically referred to as "astaxanthins") having antioxidant effects, anti-inflammatory effects, skin antiaging effects, whitening ability and the like and known as yellow to red colorants.

Astaxanthin is a red pigment having absorption maximum at 476 nm (ethanol) and 468 nm (hexane), and belongs to exanthophylls as one kind of carotenoid (Davies, B. H.: In "Chemistry and Biochemistry of Plant Pigments", T. W. Goodwin ed., 2nd ed., 38-165, Academic Press, NY, 1976). The chemical structure of astaxanthin is 3,3'-dihydroxy-$\beta,\beta$-carotene-4,4'-dione ($C_{40}H_{52}O_4$, molecular weight 596.82).

In the astaxanthin, three isomers of 3S,3S'-form, 3S,3R'-form (meso form) and 3R,3R'-form are present depending on a steric configurations of hydroxyl groups at 3 (3')-position of a ring structure present at both ends of the molecule. Additionally, cis- and trans-isomers of a conjugated double bond at the molecular center are present. For example, there are all cis- and 9-cis and 13-cis forms of isomer.

The hydroxyl group at a 3(3')-position can form an ester with fatty acid. Astaxanthin obtained from Euphausiacea are 3S,3S'-form when obtained from a diester having two fatty acids bonded thereto (Yamaguchi, K., Miki, W., Toriu, N., Kondo, Y., Murakami, M., Konosu, S., Satake, M., and Fujita, T.: The composition of carotenoid pigments in the antrarctic krill Euphausia superba, Bull. Jap. Sos. Sci. Fish., 1983, 49, p.1411-1415), H. pluvialis, and a large amount of a monoester having one fatty acid bonded thereto is contained (Renstrom, B., Liaaen-Jensen, S.: Fatty acids of some estrified carotenols, Comp. Biochem. Physiol. B, Comp. Biochem., 1981, 69, p.625-627).

Furthermore, astaxanthin obtained from Rhaffia Rhodozyma is 3R,3R'-form (Andrewes, A.G., Starr, M. P.: (3R,3'R)-Astraxanthin from the yeast Phaffarhodozyma, Phytochem., 1976, 15, p.1009-1011), and has a structure opposite 3S,3S'-form generally found naturally. This is present in a free form which does not form an ester with fatty acid (Andrewes, A.G., Phaffia, H. J., Starr, M. P.: Carotenids of Phaffia rhodozyma, a red pigmented fermenting yeast, Phytochem., 1976, 15, p.1003-1007).

Astaxanthin and its ester form were first separated from a lobster (Astacus gammarus L.) by Kuhn et al., and its estimated structure was disclosed (Kuhn, R., Soerensen, N. A.: The coloring matters of the lobster (Astracus gammarus L.), Z. Angew. Chem., 1938, 51, p.465-466). Since then, it has been clarified that astaxanthin is widely distributed in nature, is generally present as in the astaxanthin fatty acid ester form, and is also present as astaxanthin protein (ovorubin and crustacyanin) bonded to protein in crustacean (Cheesman, D. F.: Ovorubin, a chromoprotein from the eggs of the gastropod mollusc Pomacea canaliculata, Proc. Roy. Soc. B, 1958, 149, p.571-587).

The astaxanthin and/or its ester (astaxanthins) may be contained in the emulsion composition of the invention as an astaxanthin-containing oil separated and extracted from natural products containing astaxanthin and/or containing an astaxanthin ester. Examples of such an astaxanthin-containing oil include extracts obtained by culturing red yeast Phaffia, green alga Haematococcus, marine bacteria or the like and extracting from its culture, and extracts from antactic Euphausiaceaand the like.

It is known that Haematococcus alga extract (Haematococcus alga-derived pigment) differs from Euphausiacea-derived pigment and synthesized astaxanthin in the kind ester and its content.

Astaxanthins that can be used in the invention may be the above-described extracts, products by appropriate purification of those extracts according to need, and synthetic products. Astaxanthins that are particularly preferably, from the point of view of quality and productivity, are products extracted from Haematococcus alga (called Haematococcus alga extracts).

Specific examples that may be given of products derived from Haematococcus alga extract that can be used in the invention include Haematococcus pluvialis, Haematococcus lacustris, Haematococcus capensis, Haematococcus droebakensis and Haematococcus zimbabwiensis.

Various methods may be employed at the culture method of Haematococcus alga that can be used in the invention such as disclosed in, for example, JP-A-8-103288, the method is not particularly limited, as long as the form is changed from that of a vegetative cell to that of a cyst cell as dormant cell.

Haematococcus alga extracts that can be used in the invention are obtained by crushing, as required, cell walls of the above raw materials by a method described in, for example, JP-A No. 5-68585 and adding an extracting solvent such as an organic solvent such as acetone, ether, chloroform and alcohol (ethanol, methanol and the like), or carbon dioxide in a supercritical state, followed by extraction.

In the invention, commercially available Haematococcus alga extracts can be used, and examples thereof include ASTOTS-S, ASTOTS-2.5 O, ASTOTS-5 O and ASTOTS-10 O, products of Takedashiki Co., Ltd.; AstaREAL oil 50F and AstaREAL oil 5F, products of Fuji Chemical Industry Co., Ltd.; and BioAstin SCE7, a product of Toyo Koso Kagaku Co., Ltd.

The content of the astaxanthins as a pigment pure component in Haematococcus alga extracts that can be used in the invention is preferably from 0.001% to 50% by mass, and more preferably from 0.01% to 25% by mass, from the standpoint of handling properties in the production of the emulsion composition.

Haematococcus alga extracts that can be used in the invention contain astaxanthin or its ester as a pigment pure component similar to the pigment described in JP-A-2-49091, and contain the ester in an amount of generally 50% by mole or more, preferably 75% by mole or more, and more preferably 90% by mole or more.

Further detailed explanation is described in internet (URL:http://www.astraxanthin.co.jp/chemical/basic.htm), 2005.

(Oils)

Examples of the oils and fats in the oil-based component include oils and fats (fatty oils) that are liquid at ordinary temperature, and solid oils and greases (fats) that are solid at ordinary temperature.

Examples that may be given of liquid oils and fats include olive oil, camellia oil, macadamia nut oil, castor oil, avocado oil, evening primrose oil, turtle oil, corn oil, mink oil, rapeseed oil, egg yolk oil, sesame oil, persic oil, wheat germ oil, sasanqua oil, linseed oil, sunflower oil, cotton seed oil, perilla oil, soybean oil, peanut oil, tea seed oil, kaya oil, rice bran oil, china wood oil, tung oil, hohoba oil, germ oil, triglycerin, glycerin trioctanoate, glycerin triisopalmitate, salad oil, sunflower oil (safflower oil), palm oil, coconut oil, peanut oil, almond oil, hazelnut oil, walnut oil and grape seed oil.

Examples that may be given of the solid oils and fats include beef tallow, hydrogenated beef tallow, hoof oil, beef bone oil, mink oil, egg yolk oil, lard, horse fat, mutton tallow, hydrogenated oil, cacao oil, coconut oil, hydrogenated coconut oil, palm oil, palm hydrogenated oil, Japan tallow, Japan tallow kernel oil and hydrogenated castor oil.

Of the above, coconut oil that is a medium-chain triglyceride is preferably used from the standpoints of particle size and stability of the emulsion composition.

In the invention, commercially available products can be used as the oils and fats. Furthermore, in the invention, the oils and fats can be use alone or as mixtures thereof.

(Radial trapping agent)

The oil-based component in the invention preferably contains a lipid-soluble radical trapping agent (antioxidant) having a radical trapping function.

It is the preferred embodiment that the radial trapping agent is used alone as an oil-based component, and is used as a mixture in order to prevent oxidation of other oil-based component.

The radical trapping agent is an additive that suppresses generation of radicals, and further has a role of rapidly trapping radicals, thereby stopping a chain reaction from taking place (Source: Oil Chemistry Handbook, 4th edition, Japan Oil Chemists' Association, 2001).

Known methods of directly confirming the function as a radical trapping agent include a method in which the radical trapping agent is mixed with a reagent, and the state of trapping the radial is measured with a spectrophotomer or by ERS (electron spin resonance). Those methods use DPPH (1,1-diphenyl-2-picrylhydrazyl) or a garbinoxyl radial as the reagent.

In the invention, a compound that gives a time required to increase the peroxide value (POV) of an oil or fat to 60 meq/kg, utilizing an auto-oxidation reaction of oil and fat under the following experimental conditions, that is 2 times by weight or more that without the agent is defined as a "radial trapping agent". The peroxide value (POV) of oil or fat is measured with the conventional method.

<Conditions>

Oil or fat: Olive oil
Amount of specimen added: 0.1 % by mass based on mass of oil and fat
Test method: Sample was heated to 190°C, POV was measured with the passage of time by a conventional method, and time required to reach a POV of 60 meq/kg was calculated.

The radical trapping agent in the invention is preferably a trapping agent having a time required to reach POV of 60 meq/kg that is 5 times by weight or more that without the agent from the standpoint of stability to oxidation of an emulsion.

Compounds that can be used as the radical trapping agent of the invention may be any compound as long as it functions as a radical trapping agent in various antioxidants described in "Theory and Fact of Antioxidant" (Kajimoto, San Shobo, 1984) and "Antioxidant Handbook" (Saruwatari, Nishino and Tabata, Taiseisha, 1976), and specific examples thereof include compounds having phenolic OH, amine compounds such as phenylenediamine, and oil-solubilized derivatives of ascorbic acid and erythorbic acid.

The content of the radical trapping agent in the emulsion composition is generally from 0.001% to 20.0% by mass, preferably from 0.01% to 10% by mass, and more preferably from 0.1% to 5.0% by mass.

Examples of the preferred radical trapping agent are shown below, but the invention is limited thereto.

Examples of that may be given of compounds having phenolic OH include polyphenols (for example, catechin), guaiac gum, nordihydroguaiuretic acid (NDGA), gallic acid esters, BHT (butylhydroxytoluene), BHA (butylhydroxyanisol), vitamin Es and phenols. Examples of the gallic acid esters include propyl gallate, butyl gallate and octyl gallate.

Examples of the amine compound include phenylenediamine, diphenyl-p-phenylenediamine and 4-amino-p-diphenylamine. Diphenyl-p-phenylenediamine or 4-amino-p-diphenylamine is more preferred.

Examples of the oil-solubilized derivatives of ascorbic acid and erythorbic acid include stearic acid L-ascorbyl ester, tetraisopalmitic acid L-ascorbyl ester, palmitic acid L-ascorbyl ester, palmitic acid erythorbyl ester, and tetraisopalmitic acid erythorbyl ester.

Among the above compounds, vitamin Es are particularly preferably used from the standpoint of excellent safety and antioxidant function.

Vitamin Es are not particularly limited, and examples thereof include compounds including tocopherol and its derivative, and compounds including tocotrienol and its derivative. Those may be used alone or as mixtures thereof. Furthermore, a compound selected from the compounds including tocopherol and its derivative, and a compound selected from the compounds including tocotrienol and its derivative may be used in combination.

Examples of the compounds including tocopherol and its derivative include dl-$\alpha$-tocopherol, dl-$\beta$-tocopherol, dl-$\gamma$-tocopherol, dl-$\delta$-tocopherol, acetic acid dl-$\alpha$-tocopherol ester, nicotinic acid dl-$\alpha$-tocopherol ester, linoleic acid dl-$\alpha$-tocopherol ester and succinic acid dl-$\alpha$-tocopherol ester. Of those, dl-$\alpha$-tocopherol, dl-$\beta$-tocopherol, dl-$\gamma$-tocopherol, dl-$\delta$-tocopherol and a mixture of those (mix tocopherol) are more preferred. As the tocopherol derivative, acetic acid esters of those are preferably used.

Examples of the compounds including tocotrienol and its derivative include $\alpha$-tocotrienol, $\beta$-tocotrienol, $\gamma$-tocotrienol and $\delta$-tocotrienol. As the tocotrienol derivative, acetic acid esters of those are preferably used. The tocotrienol is a tocopherol compound analogue contained in wheals, rice bran, palm oil and the like, contains three double bonds in the side chain moiety of tocopherol, and therefore has excellent antioxidant performance.

When those vitamin Es are contained particularly in an oil phase of the emulsion composition as an oil-soluble antioxidant, the antioxidant function of an oil-based component can effectively be exhibited, and this is preferred. It is further preferred from the standpoint of the antioxidant effect that, of the above vitamin Es, at least one selected from the compounds including tocotrienol and its derivative is contained.

The content of the oil-based component in the emulsion composition of the invention is preferably from 0.1% to 50% by mass, more preferably from 0.5% to 25% by mass, and further preferably from 0.2% to 10% by mass, from the standpoints of application of the emulsion composition, emulsified particle size and emulsion stability.

When the content of the oil-based component is 0.1% by mass or more as above, the effective component is not excessively decreased, and it tends to be easy to apply the emulsion composition to foodstuff and cosmetics. When the content of the oil-based component is 50% by mass or less, there is the tendency that increases in the emulsified particle size and deterioration of the emulsion stability do not readily occur.

[Surfactant]

The surfactant used in the emulsion composition of the invention is described below. The surfactant in the invention is preferred since a surfactant emulsifying agent dissolved in an aqueous medium (hydrophilic surfactant) can greatly decrease interfacial tension between the oil phase/water phase in the emulsion composition, and as a result, the particle size can be made fine.

The surfactant in the invention has an HLB of preferably 8 or more from the standpoint of emulsion stability, more preferably 10 or more, and particularly preferably 12 or more. The upper limit of the HLB value is not particularly limited, but is generally 20 or less, and preferably 18 or less.

The HLB used herein is the balance of hydrophilicity-hydrophobicity generally used in the field of surfactants, and a generally used calculation formula g, such as Kawakami's calculation formula, can be used. The invention employs the following Kawakami's calculation formula.

$$HLB = 7 + 11.7 \log(M_w + M_o)$$

wherein $M_w$ is the molecular weight of the hydrophilic group, and $M_o$ is the molecular weight of the hydrophobic group.

HLB values described in brochures and the like may be used.

As is apparent from the above formula, a surfactant having an optional HLB value can be obtained by utilizing additive properties of HLB.

Surfactants that can be used in the invention is not particularly limited and include each of cationic, anionic, amphoteric and nonionic surfactants. However, nonionic surfactants are preferred. Examples of nonionic surfactants include glycerin fatty acid ester, organic acid monoglyceride, polygrycerin fatty acid ester, propylene glycol fatty acid ester, polyglycerin condensed licinoleic acid ester, sorbitan fatty acid ester, sucrose fatty acid ester and polyoxyethylene sorbitan fatty acid ester. Of those, polyglycerin fatty acid ester, sorbitan fatty acid ester, sucrose fatty acid ester and polyoxyethylene sorbitan fatty acid ester are preferred. The surfactant is not always required to be a surfactant that is highly purified, by distillation or the like, and may be a reaction mixture.

The polyglycerin fatty acid ester used in the invention is an ester of a polyglycerin having an average degree of polymerization of 2 or more, preferably from 6 to 15, and more preferably from 8 to 10, and a fatty acid having from 8 to 18 carbon atoms such as caprylic acid, capric acid, lauric acid, myristic acid, palmitic acid, stearic acid, oleic acid and linoleic acid. Preferred examples of the polyglycerin fatty acid ester include hexaglycerin monooleic acid ester, hexaglycerin monostearic acid ester, hexaglycerin monopalmitic acid ester, hexaglycerin monomyristic acid ester, hexaglycerin monolauric acid ester, decaglycerin monooleic acid ester, decaglycerin monostearic acid ester, decaglycerin monopalmitic acid ester, decaglycerin monomyristic acid ester and decaglycerin monolauric acid ester.

Of those, decaglycerin monooleic acid ester (HLB=12), decaglycerin monostearic acid ester (HLB=12), decaglycerin monopalmitic acid ester (HLB=13), decaglycerin monomyristic acid ester (HLB=14) and decaglycerin monolauric acid ester (HLB=16) are more preferred.

Those polyglycerin fatty acid esters can be used alone or as mixtures thereof.

Examples that may be given of suitable commercially available products include NIKKOL DGMS, NIKKOL DGMO-CV, NIKKOL DGMO-90V, NIKKOL DGDO, NIKKOL DGMIS, NIKKOL DGTIS, NIKKOL Tetraglyn 1-SV, NIKKOL Tetraglyn 1-O, NIKKOL Tetraglyn 3-S, NIKKOL Tetraglyn 5-S, NIKKOL Tetraglyn 5-O, NIKKOL Hexaglyn 1-L, NIKKOL Hexaglyn 1-M, NIKKOL Hexaglyn 1-SV, NIKKOL Hexaglyn 1-O, NIKKOL Hexaglyn 3-S, NIKKOL Hexaglyn 4-B, NIKKOL Hexaglyn 5-S, NIKKOL Hexaglyn 5-O, NIKKOL Hexaglyn PR-15, NIKKOL Decaglyn 1-L, NIKKOL Decaglyn 1-M, NIKKOL Decaglyn 1-SV, NIKKOL Decaglyn 1-50SV, NIKKOL Decaglyn 1-ISV, NIKKOL Decaglyn 1-O, NIKKOL Decaglyn 1-OV, NIKKOL Decaglyn 1-LN, NIKKOL Decaglyn 2-SV, NIKKOL Decaglyn 2-ISV, NIKKOL Decaglyn 3-SV, NIKKOL Decaglyn 3-OV, NIKKOL Decaglyn 5-SV, NIKKOL Decaglyn 5-HS, NIKKOL Decaglyn 5-IS, NIKKOL Decaglyn 5-OV, NIKKOL Decaglyn 5-O-R, NIKKOL Decaglyn 7-S, NIKKOL Decaglyn 7-O, NIKKOL Decaglyn 10-SV, NIKKOL Decaglyn 10-IS, NIKKOL Decaglyn 10-OV, NIKKOL Decaglyn 10-MAC and NIKKOL Decaglyn PR-20, products of Nikko Chemicals Co., Ltd.; RYOTO-polyglyester L-7D, L-10D, M-10D, P-8D, SWA-10D, SWA-15D, SWA-20D, S-24D, S-28D, O-15D, O-50D, B-70D, B-100D, ER-60D, LOP-120DP, DSI13W, DS3, HS11, HS9, TS4, TS2, DL15 and D013, products of Mitsubishi-Kagaku Foods Corporation; SUNSOFT Q-17UL, SUNSOFT Q-14S and SUNSOFT A-141C, products of Taiyo Kagaku Co., Ltd.; and POEM DO-100 and POEM J-0021, products of Riken Vitamin Co., Ltd.

Of the above, NIKKOL Decaglyn 1-L, NIKKOL Decaglyn 1-M, NIKKOL Decaglyn 1-SV, NIKKOL Decaglyn 1-50SV, NIKKOL Decaglyn 1-ISV, NIKKOL Decaglyn 1-O, NIKKOL Decaglyn 1-OV, NIKKOL Decaglyn 1-LN, and RYOTO-polyglyester L-7D, L-10D, M-10D, P-8D, SWA-10D, SWA-15D, SWA-20D, S-24D, S-28D, O-15D, O-50D, B-70D, B-100D, ER-60D and LOP-120DP are preferred.

The sorbitan fatty acid ester used in the invention has preferably 8 or more carbon atoms, and more preferably 12 or more carbon atoms, in the fatty acid moiety. Preferred examples of the sorbitan fatty acid ester include sorbitan monocaprylate, sorbitan monolaurate, sorbitan monostearate, sorbitan sesquistearate, sorbitan tristearate, sorbitan isotearate, sorbitan sesquiisostearate, sorbitan oleate, sorbitan sesquioleate and sorbitan trialeate.

Those sorbitan fatty acid esters can be used alone or as mixtures thereof.

Examples that may be given of suitable commercially available products of the sorbitan fatty acid ester include NIKKOL SL-10, SP-10V, SS-10V, SS-10MV, SS-15V, SS-30V, SI-10RV, SI-15RV, SO-10V, SO-15MV, SO-15V, SO-30V, SO-10R, SO-15R, SO-30R and SO-15EX, products of Nikko Chemicals Co., Ltd.; SORGEN 30V, 40V, 50V, 90 AND 110, products of Daiichi-Kogyo Seiyaku Co., Ltd.; and REODOL AS-10V, AO-10V, AO-15V, SP-L10, SP-P10, SP-S10V, SP-S30V, SP-O10V and SP-O30V, products of Kao Corporation.

The sucrose fatty acid ester used in the invention has preferably 12 or more, and more preferably from 12 to 20, carbon atoms in the fatty acid moiety.

Preferred examples of the sucrose fatty acid ester include sucrose oleic acid diester, sucrose stearic acid diester, sucrose palmitic acid diester, sucrose myristic acid diester, sucrose lauric acid diester, sucrose oleic acid monoester, sucrose stearic acid monoester, sucrose palmitc acid monoester, sucrose myristic acid monoester and sucrose lauric acid monoester. Of those, sucrose oleic acid monoester, sucrose stearic acid monoester, sucrose palmitc acid monoester, sucrose myristic acid monoester and sucrose lauric acid monoester are more preferred.

In the invention, those sucrose fatty acid esters can be used alone or as mixtures thereof.

Examples that may be given of suitable commercially available product of the sucrose fatty acid ester include RYOTO-sugar ester S-070, S-170, S-270, S-370, S-370F, S-570, S-770, S-970, S-1170, S-1170F, S-1570, S-1670, P-070, P-170, P-1570, P-1670, M-1695, O-170, O-1570, OWA-1570, L-195, L-595, L-1695, LWA-1570, B-370, B-370F, ER-190, ER-290 and POS-135, products of Mitsubishi-Kagaku Foods Corporation; DK ester SS, F160, F140, F110, F90, F70, F50, F-A50, F-20W, F-10 and F-A10E, and COSMELIKE B-30, S-10, S-50, S-70, S-110, S-160, S-190, SA-10, SA-50, P-10, P-160, M-160, L-10, L-50, L-160, L-150A, L-160A, R-10, R-20, O-10 and O-150, products of Daiichi-Kogyo Seiyaku Co., Ltd.

Of the above, RYOTO-sugar ester S-1170, S-1170F, S-1570, S-1670, P-1570, P-1670, M-1695, O-1570 and L-1695; DK ester SS, F160, F140 and F110; and COSMELIKE S-110, S-160, S-190, P-160, M-160, L-160, L-150A, L-160A and O-150 are preferred.

The polyoxyethylene sorbitan fatty acid ester used in the invention has preferably 8 or more, and more preferably 12 or more, carbon atoms in the fatty acid moiety. Furthermore, the length (number of moles added) of ethylene oxide of the polyethylene is preferably from 2 to 100, and more preferably from 4 to 50.

Preferred examples of the polyoxyethylene sorbitan fatty acid ester include polyoxyethylene sorbitan caprylic acid monoester, polyoxyethylene sorbitan lauric acid monoester, polyoxyethylene sorbitan stearic acid monoester, polyoxyethylene sorbitan stearic acid sesquiester, polyoxyethylene sorbitan stearic acid triester, polyoxyethylene sorbitan isostearic acid ester, polyoxyethylene sorbitan isostearic acid sesquiester, polyoxyethylene sorbitan oleic acid ester, polyoxyethylene sorbitan oleic acid sesquiester and polyoxyethylene sorbitan oleic acid triester.

Those polyoxyethylene sorbitan fatty acid esters can be use alone or as mixtures thereof.

Examples that may be given of suitable commercially available products of the polyoxyethylene sorbitan fatty acid ester include NIKKOL TL-10, NIKKOL TP-10V, NIKKOL TS-10V, NIKKOL TS-10MV, NIKKOL TS-106V, NIKKOL TS-30V, NIKKOL TI-10V, NIKKOL TO-10V, NIKKOL TO-10MV, NIKKOL TO-106V and NIKKOL TO-30V, products of Nikko Chemicals Co., Ltd.; REODOL TW-L106, TW-L120, TW-P120, TW-S106V, TW-S120V, TW-S320V, TW-O106V, TW-O120V, TW-O320V and TW-IS399C, and REODOL SUPER SP-L10 and TW-L120, products of Kao Corporation; and SORGEN TW-20, TW-60V and TW-80V, products of Daiichi-Kogyo Seiyaku Co., Ltd.

The amount of the surfactant in the emulsion composition of the invention is required to be an amount exceeding 0.5 times by weight the amount of the oil-based component and exceeding 5 times by weight the amount of the phospholipid. When the amount of the surfactant is more than 0.5 times by weight the amount of the oil-based component, an emulsion having a fine particle size may be obtained. When the amount is more than 5 times by weight the amount of the phospholipid, emulsion stability is not impaired. Such an amount of the surfactant can make the emulsion stability remarkably good, particularly when ascorbic acid, citric acid and/or their salts are present with the composition of the invention.

The amount o the surfactant is required to exceed 0.5 times by weight the amount of the oil-based component in order to obtain a fine particle size, but the amount preferably does not exceed 1.5 times by weight, and particularly preferably does not exceed 1 time by weight. An amount of the surfactant that does not exceed 2 times by weight is required in view that problems of heavy foaming tend to be overcome.

The amount of the surfactant is required to exceed 5 times by weight the amount of the phospholipid in order to make the emulsion stability good, and the amount of the surfactant does not exceed 50 times by weight, preferably does not exceed 30 times by weight, and particularly preferably does not exceed 15 times by weight. An amount of the surfactant that does not exceed 50 times by weight is appropriate to give fine particle size and emulsion stability, and additionally there is the tendency to suppress the occurrence problems such as, for example, foaming of the composition.

The amount of the surfactant added is from 2% to 15% by mass, based on the mass of the emulsion composition.

When the amount of the surfactant is 0.5% by mass or more, it is easy to decrease interfacial tension between the oil phase/water phase.

[Polyhydric alcohol]

The emulsion composition of the invention preferably contains a polyhydric alcohol from the standpoint of particle size, stability and antiseptic property.

The polyhydric alcohol has a moisturizing function, a viscosity regulating function and the like. Furthermore, the polyhydric alcohol has the function to decrease interfacial tension between water and the oil or fat component to facilitate the interface expansion, thereby making it easy to form fine and stable particles.

From the above, it is preferred to contain a polyhydric alcohol in the emulsion composition from the standpoint that emulsion particle size can be made more fine, and the particle size can be maintained stably over a long period of time in the state of a fine particle size.

Furthermore, addition of the polyhydric alcohol can decrease water activity of the emulsion composition, thereby suppressing propagation of microorganisms.

In the invention, any polyhydric alcohol can be used, without particular limitation so long as it is a dihydric alcohol or above. Examples of the polyhydric alcohol include glycerin, diglycerin, triglycerin, polyglycerin, 3-methyl-1,3-butanediol, 1,3-butylene glycol, isoprene glycol, polyethylene glycol, 1,2-pentanediol, 1,2-hexanediol, propylene glycol, dipropylene glycol, polypropylene glycol, ethylene glycol, diethylene glycol, pentaerythritol, neopentyl glycol, multitol, reduced starch syrup, sucrose, lactitol, palatinit, erythritol, sorbitol, mannitol, xylitol, xylose, glucose, lactose, mannose, maltose, galactose, fructose, inositol, pentaerythritol, maltotriose, sorbitan, trehalose, starch degraded sugar and starch degraded sugar reduced alcohol. Those can be used alone or as mixtures of those.

Polyhydric alcohols having 3 or more hydroxyl groups in one molecule are preferably used. Use of such a polyhydric alcohol can further effectively decrease interfacial tension between an aqueous solvent and the oil or fat component, making it possible to form fine and stable particles. As a result, in the use in foodstuff, intestinal absorption properties can be increased further, and in the use in cosmetics, percutaneous absorption properties can be increased further.

When polyhydric alcohols satisfying the above-described conditions are used, particularly when glycerin is used, the particle size of the emulsion is further decreased, the particle size is maintained stably over a long period of time while keeping a fine particle size, and therefore are preferred.

The content of the polyhydric alcohol is preferably from 10% to 60% by mass, more preferably 20% to 55% by mass, and further preferably from 30% to 50% by mass, based on the mass of the emulsion composition, from the standpoint of viscosity of the emulsion composition in addition to the above-described particle size, stability and preservative properties.

When the content of the polyhydric alcohol is 10% by mass or more, it is preferred, from the standpoint that it is easy to obtain sufficient storage stability, even when the kind and content of the oil or fat component, and the like varies. On the other hand, it is preferred that the content of the polyhydric alcohol is 60% by mass or less, from the standpoints that the maximum effect is obtained, and it is easy to suppress increases in the viscosity of the emulsion composition.

According to need, other additives can be added to the emulsion composition of the invention.

The particle size of the emulsion composition is not particularly limited, but is preferably 200 nm or less, more preferably 150 nm or less, and most preferably 90 nm or less.

When the particle size of the emulsion composition is 200 nm or less, it is preferred from the standpoint that the transparency of foodstuff, cosmetics and the like produced using such an emulsion do not readily deteriorate, and systematic and percutaneous absorption properties do not readily deteriorate.

The particle size of the emulsion composition of the invention can be measured with a commercially available particle size distribution measuring device. Optical microscopy, confocal laser microscopy, electron microscopy, atomic force microscopy, static light scattering method, laser diffraction method, dynamic light scattering method, centrifugal precipitation method, electric pulse measurement method, chromatography method, ultrasonic damping method and the like are known as particle size distribution measurement methods of an emulsion, and devices corresponding to the respective principle are commercially available.

From particle size range in the invention and ease of measurement, a dynamic light scattering method is preferred in the emulsion particle size measurement of the invention. Commercially available measurement devices using dynamic light scattering include Nanotrac UPA (Nikkiso Co., Ltd.), dynamic light scattering particle size distribution measuring device LB-550 (Horiba, Ltd.) and fiber-optics particle size analyzer FPAR-1000 (Otsuka Electronics Co., Ltd.).

The particle size in the invention is a value measured using the above-described dynamic light scattering particle size distribution measuring device LB-550 (Horiba, Ltd.), and specifically employs the value measured as follows.

Measurement method of the particle size is as follows. Dilution is conducted with pure water such that the concentration of an oil-based component is 1% by mass. Measurement is conducted using a quartz cell. The particle size is derived as the median size when the refractive index of a sample is set to 1.600, the refractive index of the dispersion medium is set to 1.333 (pure water) and the viscosity of a dispersion medium is set to the viscosity of pure water.

The particle size of the emulsion composition can be made fine, other than by using the components of an emulsion composition as described before, by the factors such as the stirring conditions (shear force, temperature and pressure) in the production method of an emulsion composition described hereinafter, the oil phase/water phase ratio, and the like.

<Production method of emulsion composition>

The production method of the emulsion composition of the invention is not particularly limited. However, for example, a preferred production method includes steps of a) dissolving a surfactant in an aqueous medium (water or the like) to obtain a water phase, b) mixing and dissolving the above-described oil-based component (carotenoid or the like) and phospholipid (lecithin or the like) to obtain an oil phase, and c) mixing the water phase and the oil phase under stirring to carry out emulsion dispersion, thereby obtaining an emulsion composition.

Components contained in the oil phase and water phase in the above production method are the same as the constituents of the emulsion composition of the invention described before, and the preferred examples and the preferred amounts are the same, and the preferred combinations are more preferable.

The ratio (mass) of the oil phase to the water phase in the emulsification dispersion is not particularly limited, but the oil phase:water phase ratio (% by mass) is preferably from 0.1:99.9 to 50:50, more preferably from 0.5:99.5 to 30/70, and further preferably from 1:99 to 20:80.

An oil phase:water phase ratio of 0.1:99.9 or more is preferred, since the effective components are not too low, there is the tendency that practical problems with the emulsion composition do not readily occur. An oil phase/water phase ratio of 50:50 or less is preferred, since the concentration of the surfactant is not too low, and there is the tendency that emulsion stability of the emulsion composition does not deteriorate.

The emulsification dispersion may be sufficient by carrying out the emulsification operation in one step, but it is preferable to carry out emulsion operation in two steps or more, from the point of obtaining uniform and fine emulsified particles.

Specifically, it is particularly preferable that two kinds or more of emulsification device are used in combination, by the method of emulsifying with a high pressure homogenizer, ultrasonic dispersing machine or the like, in addition to the emulsification operation of one step of emulsifying using a general emulsification apparatus (for example, stirrer, impeller stirring, homomixer, continuous-flow shearing apparatus or the like) utilizing shear action. When a high pressure homogenizer is used, the emulsion may be formed with liquid droplets of more uniform fine particles. Furthermore, emulsification dispersion may be conducted repeatedly for the purpose of forming liquid droplets having more uniform particle sizes.

The emulsion composition of the invention can be widely used in foodstuff or cosmetics. The foodstuff used herein include beverages and frozen desserts, and cosmetics used herein include skin cosmetic (lotion, essence, milky lotion, cream and the like), lipsticks, sun block cosmetics and makeup cosmetics. However, foodstuff and cosmetics are not limited to those.

The foodstuff or cosmetics of the invention contain the emulsion composition of the invention. According to need, components that can be added to foodstuff and cosmetics can appropriately be added to the foodstuff or the cosmetics of the invention.

The addition amount of the emulsion composition of the invention used in foodstuff and cosmetics varies depends on the type and purpose of products and cannot unconditionally be specified, but the emulsion composition can be used in a range of from 0.01% to 10% by mass, and preferably from 0.05% to 5% by mass, based on the mass of the product.

Where the addition amount is too small, the desired effect may not always be exhibited. Where the addition amount is too large, the excessive emulsion composition added may not always impart the exhibited effect.

By using the emulsion composition of the invention particularly in beverages (in the case of foodstuff), and aqueous products such as lotion, essence, milky lotion, cream pack and mask, facial mask, shampoo cosmetic, fragrance cosmetic, liquid body cleansing preparation, UV care cosmetic, deodorant cosmetic and oral care cosmetic (in the case of cosmetics), products having a transparent feeling are obtained, and the occurrence of disadvantageous phenomena, such as precipitation, sedimentation or choker ring of insoluble matters under severe conditions, such as long-term storage or sterilization treatment, may be suppressed.

The foodstuff or cosmetics of the invention can be obtained by, for example, mixing the emulsion composition of the invention and according to need, components that can be added, by the conventional methods.

EXAMPLES

The present invention is specifically explained by way of Examples below, which should not be construed as limiting the invention thereto.

(Example 1)

The following components were dissolved with heating at 70°C for 1 hour to obtain a water phase composition.

| | |
|---|---|
| Sucrose stearic acid ester (HLB=16) | 33.0 g |
| Decaglyceryl monooleate (HLB=12) | 67.0 g |
| Glycerin | 450.0 g |

(continued)

| | |
|---|---|
| Pure water | 300.0 g |

The following components were dissolved under heating at 70°C for 1 hour to obtain an oil phase composition.

| | |
|---|---|
| Haematococcus agla extract | 37.5 g |
| (content of astaxanthins: 20% by mass) | |
| Mix tocopherol | 9.5 g |
| Coconut oil | 93.0 g |
| Lecithin (derived from soybean) | 10.0 g |

The above water phase composition was stirred (10,000 rpm) with a homogemizer (Model HP93, a product of SMT Co.) maintaining at 70°C, and the above oil phase composition was added thereto to obtain an emulsion.
Subsequently, the pre-emulsion obtained was cooled to about 40°C, and was subjected to high pressure-emulsification under a pressure of 200 MPa using an ULTIMIZER HJP-25005 (a product of Sugino Machine Limited).
The emulsion product was then filtered with a microfilter having an average pore size of 1 μm to prepare an astaxanthin-containing emulsion composition EM-01.
Astaxanthin-containing emulsion compositions EM-02 to 10 were obtained in the same manner as above, except for changing the composition as shown in Table 1 below.

[Table 1]

| | | EM-01 Invention | EM-02 Invention | EM-03 Invention | EM-06 Invention | EM-07 Invention | EM-08 Comparison | EM-09 Comparison | EM-10 Comparison |
|---|---|---|---|---|---|---|---|---|---|
| Water phase | Sucrose stearic acid ester (HLB=16) | 33.0 | 60.0 | 60.0 | 33.0 | - | 16.5 | 20.0 | - |
| | Sucrose stearic acid ester (HLB=7) | - | - | - | - | 33.0 | - | - | - |
| | Sucrose lauric acid ester (HLB=16) | - | - | - | - | - | - | - | 43.0 |
| | Decaglyceryl monooleate (HLB=12) | 67.0 | 60.0 | 60.0 | 67.0 | - | 33.5 | 40.0 | - |
| | Hexaglyceryl monooleate (HLB=9) | - | - | - | - | 67.0 | - | - | - |
| | Decaglyceryl monolaurate (HLB=15.5) | - | - | - | - | - | - | - | 71.0 |
| | Glycerin | 450.0 | 630.0 | 630.0 | 450.0 | 450.0 | 450.0 | 450.0 | 286.0 |
| | Pure water | 300.0 | 160.0 | 160.0 | 300.0 | 300.0 | 350.0 | 344.0 | 457.0 |
| Oil phase | Haematococcus alga extract | 37.5 | 37.5 | 37.5 | 37.5 | 37.5 | 37.5 | 37.5 | 37.5 |
| | Mix tocopherol | 9.5 | 1.0 | 1.0 | 9.5 | 9.5 | 9.5 | 9.5 | - |
| | Coconut oil | 93.0 | 46.5 | 46.5 | 93.0 | 93.0 | 93.0 | 93.0 | 105.5 |
| | Lecithin | 10.0 | 5.0 | - | - | 10.0 | 10.0 | 6.0 | - |
| | Lysolecithin | - | - | 5.0 | - | - | - | - | - |
| | Sphingomyelin | - | - | - | 10.0 | - | - | - | - |
| Total | | 1000 | 1000 | 1000 | 1000 | 1000 | 1000 | 1000 | 1000 |

EP 1 927 287 B1

12

In Table 1, the sucrose stearic acid ester used was RYOTO-sugar ester S-1670 (HLB=16) and S-770 (HLB=7), products of Mitsubishi-Kagaku Foods Corporation; the sucrose lauric acid ester used was RYOTO-sugar ester L-1695 (HLB=16), a product of Mitsubishi-Kagaku Foods Corporation; the decaglyceryl monooleate used was NIKKOL Decaglyn 1-O (HLB=12), a product of Nikko Chemicals Co., Ltd.; the hexaglyceryl monooleate used was NIKKOL Hexaglyn 1-OV (HLB=9), a product of Nikko Chemicals Co., Ltd.; and the decaglyceryl monolaurate used was NIKKOL Decaglyn 1-L (HLB=15.5), a product of Nikko Chemicals Co., Ltd. The Haematococcus extract used was ASTOTS-S, a product of Takeda Shiki Co., Ltd. The mix tocopherol used was RIKEN E OIL 800, a product of Riken Vitamin Co., Ltd. The coconut oil used was COCONAD MT, a product of Kao Corporation. The lecithin (derived from soybean) used was LECION P, a product of Riken Vitamin Co., Ltd. The lysolecithin used was LECIMAL EL, a product of Riken Vitamin Co., Ltd. The sphingomyelin used was COATSOME NM-70, a product of NOF Corporation.

(Measurement of particle size)

1.0 g of each of the astaxanthin-containing emulsion compositions (EM-01 to 10) obtained above was added to 99.0 g of pure water, and stirring was conducted for 10 minutes using a magnetic stirrer. A particle size of a water-diluted solution of the emulsion composition obtained was measured using a dynamic light scattering particle size dispersion measuring device LB-550 (a product of Horiba, Ltd.). The results are shown in Table 2 below.
The specific measurement method is the same as described before.

(Emulsion stability)

As emulsion stability, the increase of particle size when ascorbic acid sodium salt was added, or was not added, was examined.
10 g of ascorbic acid sodium salt (powder) was added to 90 g of a water-diluted solution of the above emulsion composition, and stirring was conducted for 10 minutes using a stirrer. A particle size of the aqueous solution obtained was measured using LB-550 in the same manner as above.
Similarly, trisodium citrate was added in place of ascorbic acid sodium salt, and the particle size was measured.
Similarly, 1 g of catechin was added in place of 10 g of ascorbic acid sodium salt (powder), and the particle size was measured. Catechin used was PF-TP80, a product of Pharma Foods International Co., Ltd. The results are shown in Table 2 below.

[Table 2]

| | | EM-01 Invention | EM-02 Invention | EM-03 Invention | EM-06 Invention | EM-07 Invention | EM-08 Comparison | EM-09 Comparison | EM-10 Comparison |
|---|---|---|---|---|---|---|---|---|---|
| Surfactant/oil-based component Surfactant/phospho lipid | | 0.71 10 | 1.41 24 | 1.41 24 | 0.71 10 | 0.71 10 | 0.36 5 | 0.43 10 | 0.8 - |
| Kind of phospholipid | | Lecithin | Lecithin | Lysolecithin | Sphingomyelin | Lecithin | Lecithin | Lecithin | Not used |
| Particle size | Pure water dilution | 58 nm | 56 nm | 96 nm | 61 nm | 102 nm | 81 nm | 70 nm | 60 nm |
| | Ascorbic acid Na addition | 62 nm | 60 nm | 107 nm | 76 nm | 110 nm | 115 nm | 104 nm | 160 nm |
| | Trisodium citrate addition | 65 nm | 63 nm | 117 nm | 79 nm | 123 nm | 168 nm | 146 nm | Agglome-ration |
| | Catechin addition | 121 nm | 127 nm | 237 nm | 147 nm | 221 nm | 344 nm | 290 nm | Agglome-ration |

EP 1 927 287 B1

As is apparent from Table 2 above, it is seen that the emulsion composition of the invention has a fine particle size, and shows very excellent emulsion stability, even when an organic acid (salt) such as ascorbic acid sodium salt, and polyphenols such as catechin are added.

(Example 2)

The following components were dissolved with heating at 70°C for 1 hour to obtain a water phase composition.

| | |
|---|---|
| Sucrose stearic acid ester (HLB=16) | 33.0 g |
| Decaglyceryl monooleate (HLB=12) | 67.0 g |
| Glycerin | 450.0 g |
| Pure water | 300.0 g |

The following components were dissolved under heating at 70°C for 1 hour to obtain an oil phase composition.

| | |
|---|---|
| Haematococcus agla extract | 37.5 g |
| (content of astaxanthins: 20% by mass) | |
| Tocotrienol-containing oil | 9.5 g |
| (content of tocotrienol: 50% by mass) | |
| Coconut oil | 93.0 g |
| Lecithin (derived from soybean) | 10.0 g |

The above water phase composition was stirred (10,000 rpm) with a homogemizer (Model HP93, a product of SMT Co.) maintaining at 70°C, and the above oil phase composition was added thereto to obtain an emulsion.

Subsequently, the pre-emulsion obtained was cooled to about 40°C, and was subjected to high pressure-emulsification under a pressure of 200 MPa using ULTIMIZER HJP-25005 (a product of Sugino Machine Limited).

The emulsion was then filtered with a microfilter having an average pore size of 1 $\mu$m to prepare astaxanthins-containing emulsion composition EM-21.

Astaxanthins-containing emulsion compositions EM-22 to 24 were obtained in the same manner except for changing the composition as shown in Table 3 below. EM-23 corresponds to EM-01 in Example 1.

In the Table, the sucrose stearic acid ester used was RYOTO-sugar ester S-1670 (HLB=16), a product of Mitsubishi-Kagaku Foods Corporation, and the decaglyceryl monooleate used was NIKKOL Decaglyn 1-O, a product of Nikko Chemicals Co., Ltd. The Haematococcus extract used was ASTOTS-S, a product of Takeda Shiki Co., Ltd. The tocotrienol-containing oil used was TOCOMIN 50%, a product of Carotec Co., Ltd. The mix tocopherol used was RIKEN E OIL 800, a product of Riken Vitamin Co., Ltd. The coconut oil used was COCONAD MT, a product of Kao Corporation. The lecithin (derived from soybean) used was LECION P, a product of Riken Vitamin Co., Ltd.

[Table 3]

| | | EM-21 Invention | EM-22 Invention | EM-23 Invention | EM-24 Invention |
|---|---|---|---|---|---|
| Water phase | Sucrose stearic acid ester (HLB=16) | 33.0 g | 33.0 g | 33.0 g | 33.0 g |
| | Decaglyceryl monooleate (HLB=12) | 67.0 g | 67.0 g | 67.0 g | 67.0 g |
| | Glycerin | 450.0 g | 450.0 g | 450.0 g | 450.0 g |
| | Pure water | 300.0 g | 300.0 g | 300.0 g | 300.0 g |
| Oil phase | Haematococcus agla extract | 37.5 g | 37.5 g | 37.5 g | 37.5 g |
| | Tocotrienol-containing oil | 9.5 g | - | - | - |
| | Mix tocopherol | - | 4.8 g | 9.5 g | - |
| | Coconut oil | 93.0 g | 97.7 g | 93.0 g | 102.5 g |
| | Lecithin | 10.0 g | 10.0 g | 10.0 g | 10.0 g |

(continued)

| | EM-21 Invention | EM-22 Invention | EM-23 Invention | EM-24 Invention |
|---|---|---|---|---|
| Total | 1000 g | 1000 g | 1000 g | 1000 g |

(Dilution)

1.0 g of each of the astaxanthin-containing emulsion compositions (EM-21 to 24) obtained was added to 99.0 g of pure water, and stirring was carried out with a magnetic stirrer for 5 minutes. The thus-obtained water-diluted solutions of the emulsion compositions were evaluated as follows. The results are shown in Table 4 below.
Particle size measurement and emulsion stability were according to the same manner as in Example 1.

(Evaluation of decomposition stability of astaxanthins)

Absorbance (Ci) of a water-diluted solution of an emulsion composition was measured using ND-1000 Spectrophotometer, a product of NanoDrop Technologies, Inc. A water-diluted solution of an emulsion composition was placed in a glass bottle with a cap, and the glass bottle was stored in a thermostat chamber maintained at 50°C. Storage days were 1, 3, 5, 7, 10 and 14. After the storage of the respective days, absorbance was measured to obtain absorbance (Cf) after the storage, and an astaxanthin residual ratio = Cf/Ci x 100 was obtained.
A graph was prepared such that the horizontal axis is the storage day and the vertical axis is the astaxanthin residual ratio, the number of storage days when the astaxanthin residual ratio reaches 80% was read off from the graph, and the value was evaluated as 80% astaxanthin residual days.

[Table 4]

| | | EM-21 Invention | EM-22 Invention | EM-23 Invention | EM-24 Invention |
|---|---|---|---|---|---|
| Tocotrienol content (% by mass) | | 0.48 | 0 | 0 | 0 |
| Tocopherol content (% by mass) | | 0 | 0.48 | 0.95 | 0 |
| Particle size | Pure water dilution | 55 nm | 55 nm | 57 nm | 53 nm |
| | Ascorbic acid Na addition | 58 nm | 57 nm | 60 nm | 56 nm |
| | Trisodium citrate addition | 61 nm | 59 nm | 64 nm | 57 nm |
| | Catechin addition | 114 nm | 112 nm | 119 nm | 110 nm |
| 80% astaxanthin residual days (day) | | 13 | 10 | 11 | 1 |

Particle size of oil droplets after dilution with pure water and particle size thereof after addition of an organic acid were measured in the emulsion compositions EM-21, EM-22, EM-23 and EM-24 according to the invention in the same manner as in Example 1. The results are shown in Table 4.
The emulsion compositions EM-21 to EM-24 according to the invention each showed good emulsion stability without appearing agglomeration and the like even when an organic acid (salt) such as ascorbic acid sodium salt, and polyphenols such as catechin were added, similarly to EM-01 of Example 1.
Furthermore, as is apparent from Table 4, of the astaxanthin-containing emulsion compositions EM-21 to EM-24 according to the invention, EM-21 to EM-23 having an oil-soluble antioxidant (oil-soluble radical trapping agent) added thereto showed high decomposition stability as compared with EM-24 which does not contain a radical trapping agent, and among those, EM-21 containing tocotrienol showed particularly good storage stability. Thus, from the fact that effective storage stability is obtained even with a smaller amount by selecting tocotrienol as the oil-soluble antioxidant, tocotrienol was found to be particularly useful when it is desired to decrease the amount of the oil-soluble antioxidant added and to increase the amount of the functional oil-based component.

(Example 3): Beverage

Beverage was prepared with the following composition and production method using the emulsion composition obtained in Example 1.

<Composition>

| | |
|---|---|
| (1) Emulsion composition of Example 1 (EM-01) | 20 g |
| (2) Fructose glucose syrup | 120 g |
| (3) Vitamin C (L-ascorbic acid) | 10 g |
| (4) Citric acid | 10 g |
| (5) Orange perfume | 3 g |
| (6) Water | 837 g |
| Total | 1,000 g |

The above components (2) to (6) were mixed and dissolved, and the component (1) was added thereto, followed by mixing, to prepare a drinkable liquid. This was filled in a bottle, and sterilized under heating at 85°C for 10 minutes. This was cooled to room temperature to obtain beverage.

The beverage obtained had excellent transparency, and occurrence of turbidity, choker ring or the like was not recognized even though the beverage was allowed to stand.

(Example 4): Skin lotion

A skin lotion was prepared with the following composition and production method using the emulsion composition obtained in Example 1.

<Composition>

| | |
|---|---|
| (1) 1,3-Butanediol | 60 g |
| (2) Glycerin | 40 g |
| (3) Oleyl alcohol | 1 g |
| (4) Polyoxyethylene (20) sorbitan monolauric acid ester | 5 g |
| (5) Polyoxyethylene (15) lauryl alcohol ether | 5 g |
| (6) Ethanol | 100 g |
| (7) Methylparaben | 2 g |
| (8) L-Ascorbic acid Na | 10 g |
| (9) Emulsion composition of Example 1 (EM-01) | 1 g |
| (10) Purified water | 776 g |
| Total | 1,000 g |

(1) was added to and dissolved in (10) to obtain a water phase. (2) to (5), (7) and (8) were dissolved in (6), and the resulting mixture was mixed with the water phase and stirred. (9) was added, followed by stirring and mixing, thereby obtaining a lotion.

The lotion obtained had excellent transparency, and occurrence of turbidity was not recognized even though the lotion was allowed to stand and stored at 50°C for 3 months.

According to the invention, an emulsion composition having small particle size of emulsified particles and excellent emulsion stability, and foodstuff and cosmetics containing the same can be provided.

Further the invention provides the following items of <1> to <14>;

<1>. An emulsion composition including a phospholipid, a surfactant and an oil-based component comprising astaxanthin or a derivative thereof, wherein the content of the surfactant exceeds 0.5 times by weight but does not exceed 2 times by weight the content of the oil-based component, and exceeds 5 times by weight but does not exceed 50 times by weight the content of the phospholipid and wherein the amount of surfactant present is 2 - 15% by mass based on the mass of the emulsion composition.

<2>. An emulsion composition of item <1>, wherein the surfactant is a nonionic surfactant having a Hydrophilic/Lipophilic Balance (HLB) of 10 or more.

<3>. An emulsion composition of any one of items <1> to <2>, wherein the surfactant is at least one of a sucrose fatty acid ester, a polyglycerin fatty acid ester, a sorbitan fatty acid ester and a polyoxyethylene sorbitan fatty acid ester.

<4>. An emulsion composition of any one of items <1> to <3>, wherein the content of the oil-based component is from 0.1% to 50% by mass based on the total mass of the composition, and the content of phospholipid is from 0.1% to 10% by mass based on the total mass of the composition.

<5>. An emulsion composition of any one of items <1> to <4>, further comprising a polyhydric alcohol.

<6>. An emulsion composition of item <5>, wherein the content of the polyhydric alcohol is from 10% to 60% by mass based on the total mass of the composition.

<7>. An emulsion composition of items <5> or <6>, wherein the polyhydric alcohol is glycerin.

<8>. An emulsion composition of any one of items <1> to <>, wherein the oil-based component comprises a lipophilic radical trapping agent.

<9>. An emulsion composition of item <8>, wherein the radical trapping agent comprises a Vitamin E.

<10>. An emulsion composition of item <8>, wherein the radical trapping agent comprises tocopherol or a derivative thereof.

<11>. An emulsion composition of item <8>, wherein the radical trapping agent comprises tocotrienol or a derivative thereof.

<12>. An emulsion composition of any one of items <1> to <11>, wherein the particle size of the emulsion is 200 nm or less.

<13>. A foodstuff comprising an emulsion composition as defined in any one of items <1> to <12>.

<14>. A cosmeticcomprising an emulsion composition as defined in any one of items <1> to <13>.

## Claims

1. An emulsion composition comprising a phospholipid, a surfactant and an oil-based component comprising astaxanthin or a derivative thereof, wherein the content of the surfactant exceeds 0.5 times by weight but does not exceed 2 times by weight the content of the oil-based component, and exceeds 5 times by weight but does not exceed 50 times by weight the content of the phospholipid and wherein the amount of surfactant present is 2-15 % by mass based on the mass of the emulsion composition.

2. An emulsion composition according to Claim 1, wherein the surfactant is a nonionic surfactant having a Hydrophilic/Lipophilic Balance (HLB) of 10 or more.

3. An emulsion composition according to Claim 1 or Claim 2, wherein the surfactant is at least one of a sucrose fatty acid ester, a polyglycerin fatty acid ester, a sorbitan fatty acid ester and a polyoxyethylene sorbitan fatty acid ester.

4. An emulsion composition according to any preceding Claim, wherein the content of the oil-based component is from 0.1 % to 50% by mass based on the total mass of the composition, and the content of phospholipid is from 0.1% to 10% by mass based on the total mass of the composition.

5. An emulsion composition according to any preceding Claim, further comprising a polyhydric alcohol.

6. An emulsion composition according to Claim 5, wherein the content of the polyhydric alcohol is from 10% to 60% by mass based on the total mass of the composition.

7. An emulsion composition according to Claim 5 or Claim 6, wherein the polyhydric alcohol is glycerin.

8. An emulsion composition according to any preceding Claim, wherein the oil-based component comprises a lipophilic radical trapping agent.

9. An emulsion composition according to Claim 8, wherein the radical trapping agent comprises a Vitamin E.

10. An emulsion composition according to Claim 8, wherein the radical trapping agent comprises tocopherol or a derivative thereof.

11. An emulsion composition according to Claim 8, wherein the radical trapping agent comprises tocotrienol or a derivative thereof.

12. An emulsion composition according to any preceding Claim, wherein the particle size of the emulsion is 200 nm or less.

13. A foodstuff comprising an emulsion composition as defined in any preceding Claim.

**14.** A cosmetic comprising an emulsion composition as defined in any preceding Claim.

**Patentansprüche**

**1.** Emulsionszusammensetzung, umfassend ein Phospholipid, ein Tensid und eine ölbasierte Komponente, die Astaxanthin oder ein Derivat davon umfasst, wobei der Gehalt des Tensids, bezogen auf das Gewicht, mehr als das 0,5-Fache, aber nicht mehr als das 2-Fache des Gehalts der ölbasierten Komponente und mehr als das 5-Fache, aber nicht mehr als das 50-Fache des Gehalts des Phospholipids beträgt und wobei die vorliegende Tensidmenge 2-15 Massen-%, bezogen auf die Masse der Emulsionszusammensetzung, beträgt.

**2.** Emulsionszusammensetzung nach Anspruch 1, wobei es sich bei dem Tensid um ein nichtionisches Tensid mit einem HLB-Wert (HLB = Hydrophilic/Lipophilic Balance) von 10 oder mehr handelt.

**3.** Emulsionszusammensetzung nach Anspruch 1 oder Anspruch 2, wobei es sich bei dem Tensid um einen Saccharosefettsäureester, einen Polyglycerinfettsäureester, einen Sorbitanfettsäureester und/oder einen Polyoxyethylensorbitanfettsäureester handelt.

**4.** Emulsionszusammensetzung nach einem der vorhergehenden Ansprüche, wobei der Gehalt der ölbasierten Komponente 0,1 bis 50 Massen- %, bezogen auf die Gesamtmasse der Zusammensetzung, beträgt und der Gehalt an Phospholipid 0,1 bis 10 Massen-%, bezogen auf die Gesamtmasse der Zusammensetzung, beträgt.

**5.** Emulsionszusammensetzung nach einem der vorhergehenden Ansprüche, die ferner einen mehrwertigen Alkohol umfasst.

**6.** Emulsionszusammensetzung nach Anspruch 5, wobei der Gehalt des mehrwertigen Alkohols 10 bis 60 Massen-%, bezogen auf die Gesamtmasse der Zusammensetzung, beträgt.

**7.** Emulsionszusammensetzung nach Anspruch 5 oder Anspruch 6, wobei es sich bei dem mehrwertigen Alkohol um Glycerin handelt.

**8.** Emulsionszusammensetzung nach einem der vorhergehenden Ansprüche, wobei die ölbasierte Komponente einen lipophilen Radikalfänger umfasst.

**9.** Emulsionszusammensetzung nach Anspruch 8, wobei der Radikalfänger Vitamin E umfasst.

**10.** Emulsionszusammensetzung nach Anspruch 8, wobei der Radikalfänger Tocopherol oder ein Derivat davon umfasst.

**11.** Emulsionszusammensetzung nach Anspruch 8, wobei der Radikalfänger Tocotrienol oder ein Derivat davon umfasst.

**12.** Emulsionszusammensetzung nach einem der vorhergehenden Ansprüche, wobei die Teilchengröße der Emulsion 200 nm oder weniger beträgt.

**13.** Lebensmittel, umfassend eine Emulsionszusammensetzung gemäß einem der vorhergehenden Ansprüche.

**14.** Kosmetikum, umfassend eine Emulsionszusammensetzung gemäß einem der vorhergehenden Ansprüche.

**Revendications**

**1.** Composition d'émulsion comprenant un phospholipide, un tensioactif et un composant à base d'huile comprenant de l'astaxanthine ou un dérivé de celle-ci, dans laquelle la teneur du tensioactif dépasse 0,5 fois en poids mais ne dépasse pas 2 fois en poids la teneur du composant à base d'huile, et dépasse 5 fois en poids mais ne dépasse pas 50 fois en poids la teneur du phospholipide et dans laquelle la quantité de tensioactif présent est de 2 à 15 % en masse sur la base de la masse de la composition d'émulsion.

**2.** Composition d'émulsion selon la revendication 1, dans laquelle le tensioactif est un tensioactif non ionique ayant un bilan hydrophile/lipophile (HLB) de 10 ou plus.

**3.** Composition d'émulsion selon la revendication 1 ou la revendication 2, dans laquelle le tensioactif est au moins l'un d'un ester d'acide gras de saccharose, un ester d'acide gras de polyglycérine, un ester d'acide gras de sorbitane et un ester d'acide gras de sorbitane polyoxyéthylénique.

**4.** Composition d'émulsion selon l'une quelconque des revendications précédentes, dans laquelle la teneur du composant à base d'huile est de 0,1 % à 50 % en masse sur la base de la masse totale de la composition, et la teneur en phospholipide est de 0,1 % à 10 % en masse sur la base de la masse totale de la composition.

**5.** Composition d'émulsion selon l'une quelconque des revendications précédentes, comprenant en outre un alcool polyhydrique.

**6.** Composition d'émulsion selon la revendication 5, dans laquelle la teneur de l'acide polyhydrique est de 10 % à 60 % en masse sur la base de la masse totale de la composition.

**7.** Composition d'émulsion selon la revendication 5 ou la revendication 6, dans laquelle l'alcool polyhydrique est la glycérine.

**8.** Composition d'émulsion selon l'une quelconque des revendications précédentes, dans laquelle le composant à base d'huile comprend un agent de piégeage de radical lipophile.

**9.** Composition d'émulsion selon la revendication 8, dans laquelle l'agent de piégeage de radical comprend une vitamine E.

**10.** Composition d'émulsion selon la revendication 8, dans laquelle l'agent de piégeage de radical comprend du tocophérol ou un dérivé de celui-ci.

**11.** Composition d'émulsion selon la revendication 8, dans laquelle l'agent de piégeage de radical comprend du tocotriénol ou un dérivé de celui-ci.

**12.** Composition d'émulsion selon l'une quelconque des revendications précédentes, dans laquelle la taille de particule de l'émulsion est de 200 nm ou moins.

**13.** Aliment comprenant une composition d'émulsion telle que défini dans l'une quelconque des revendications précédentes.

**14.** Cosmétique comprenant une composition d'émulsion telle que définie dans l'une quelconque des revendications précédentes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2078432 A
- JP 2000178130 A
- JP 2006513172 T
- JP 09157159 A
- JP 07171377 A
- WO 9528847 A
- EP 1474992 A
- JP 2006129841 B
- EP 1281396 A
- US 5248509 A
- JP 8103288 A
- JP 5068585 A
- JP 2049091 A

### Non-patent literature cited in the description

- **DAVIES, B. H.** In ''Chemistry and Biochemistry of Plant Pigments. Academic Press, 1976, 38-165
- **YAMAGUCHI, K. ; MIKI, W. ; TORIU, N. ; KONDO, Y. ; MURAKAMI, M. ; KONOSU, S. ; SATAKE, M. ; FUJITA, T.** The composition of carotenoid pigments in the antarctic krill Euphausia superba. *Bull. Jap. Sos. Sci. Fish.,* 1983, vol. 49, 1411-1415
- **RENSTROM, B. ; LIAAEN-JENSEN, S.** Fatty acids of some estrified carotenols. *Comp. Biochem. Physiol. B, Comp. Biochem.,* 1981, vol. 69, 625-627
- **ANDREWES, A.G. ; STARR, M. P.** 3R,3'R)-Astraxanthin from the yeast Phaffarhodozyma. *Phytochem.,* 1976, vol. 15, 1009-1011
- **ANDREWES, A.G. ; PHAFFIA, H. J. ; STARR, M. P.** Carotenids of Phaffia rhodozyma, a red pigmented fermenting yeast. *Phytochem.,* 1976, vol. 15, 1003-1007
- **KUHN, R. ; SOERENSEN, N. A.** The coloring matters of the lobster (Astracus gammarus L.). *Z. Angew. Chem.,* 1938, vol. 51, 465-466
- **CHEESMAN, D. F.** Ovorubin, a chromoprotein from the eggs of the gastropod mollusc Pomacea canaliculata. *Proc. Roy. Soc. B,* 1958, vol. 149, 571-587
- Oil Chemistry Handbook. Japan Oil Chemists' Association, 2001
- **KAJIMOTO.** *Theory and Fact of Antioxidant,* 1984
- **SARUWATARI ; NISHINO ; TABATA.** Antioxidant Handbook. Taiseisha, 1976